# EUROPEAN PATENT APPLICATION

(11) **EP 1 496 462 A1**
(43) Date of publication of application: **12.01.2005**
(21) Application number: 04425512.3
(22) Date of filing: 12.07.2004
(51) Int. Cl.: G06F 17/60, G06F 19/00

(54) **Centralized data processing and collecting, on the basis of the reading of the information on a paper support, preferably a medical prescription**

(30) Priority: 11.07.2003 IT rm20030339
(71) Applicant: Rinaldi, Stefano, I-00198 Roma (IT)
(72) Inventor: Rinaldi, Stefano, I-00198 Roma (IT)
(74) Representative: Iannone, Carlo Luigi

(57) **Abstract**

The present invention relates to a centralised data processing and collecting system, on the basis of the reading of the information on a paper support, preferably a medical prescription, comprising
a plurality of terminals suitable to read said paper support, extracting the written information, obliterate said paper support and memorising said information, at least one server for receiving and processing said data stored in said plurality of terminals
data from said plurality of terminals being transmitted to aid server by network connection and/or by physical transfer of data supporting means.

## Description

The present invention relates to a centralised data processing and collecting system, on the basis of the reading of the information on a paper support, preferably a medical prescription.

More specifically, the invention concerns a system of the above kind comprises a terminal particularly studied and realised for acquiring, reading and storing data during purchasing of medicine with a pharmacy, but can be used each time is necessary reading a paper support the information of which must be selected and memorised.

In the following, the specification will be addressed to the use of the system according to the invention in the sanitary and pharmacy field, but it is well evident that the same must not be considered limited to this specific use.

It is known that at present expenses for public health is an always more relevant problem for all the public administrations. In fact, control of expenses is controlled by the manual activity. However, due to the high costs of personnel of the province administrations, often it is not carried out.

In this situations, pharmacies only carry out the activity of summary of expenses and of the purchased drugs. In fact, when a prescription arrives, pharmacist puts suitable coupons on said prescription to obtain reimbursement of drugs. Then, prescriptions are sent to suitable collection centres, in numbered and organised packages, for possible inspections.

Systems exist, particularly in the US organisation, for the centralised and computerised control of the delivered drugs. However, these systems too allow to carry out *ex post* and not *ex ante* controls.

Always in the United States and in the United Kingdom, systems exist based on the previous telematic authorisation of the doctor first and of a service centre then, so as, always by telematic system, pharmacist can control the effective validity of the prescription. All the above is carried out by standard personal computers. Really, only United States of America and United Kingdom have an informatised system, but they have a health system deeply different with respect to the Italian and more generally European ones. In fact, in these countries it is provided a partial informatisation always intervening ex post, and thus with many critical points (frauds, long times, sample processing, etc.).

It would also be useful to have statistic data, e.g. relevant to the kind of drugs sold and used in determined geographic areas. This would allow to quickly localise possible viral or bacteriological epidemic, and more easily calculating their diffusion and their origin. Further, associating the name of the patient with drugs and diagnosis (in the rear part of the prescription is provided a space wherein the doctor can include the disease diagnosis), it is possible to obtain a data base of the chronic diseases or of the kind of drugs necessary to a particular person.

Finally, if it was possible to monitor the correspondence between prescriptions, ordered drugs, really sold drugs and the consumption per person of the drugs, it would be possible to carry out crossed controls allowing a general control on all the managing and contributive process at a very high level. In fact, it is very felt the problem of frauds by doctors or pharmacists ordering false prescriptions, being them aware of the lack of control or of the impossibility of coupling and crossing the data for checking.

At present, centralised systems exist allowing the collection of data after the reading of information from a paper support. Among these, it can be mentioned the system reading, collecting and sending the lottery games. However, these devices allow the reading only of pre-printed cards, possibly with pre-set spaces, but not allowing the reading of a manual writing, but some information, validating coupons, storing images of the prescriptions, putting the electronic signature and inserting them in a data base.

In view of the above, it is well evident the needing of having a system as proposed by the present invention allowing to overcome the above mentioned problems, providing a useful and practical instrument for controlling, processing and collecting data from paper supports, preferably having hand written information.

Thus, it is object of the present invention that of providing a system allowing to read the prescriptions, obtaining the number of drug boxes required and sold, obtaining possible diagnosis and being able to send these data to a centralised collection server.

Another object of the present invention is that of having a data base allowing to include all the above mentioned information to elaborate statistics and/or forecasts for the public expenses.

It is therefore specific object of the present invention a centralised data processing and collecting system, on the basis of the reading of the information on a paper support, preferably a medical prescription, comprising a plurality of terminals suitable to read said paper support, extracting the written information, obliterate said paper support and memorising said information, at least one server for receiving and processing said data stored in said plurality of terminals, data from said plurality of terminals being transmitted to aid server by network connection and/or by physical transfer of data supporting means.

Always according to the invention, each one of said plurality of terminals ca comprise at least a scanner for acquiring images from said paper support, processing means, suitable to elaborate said images of said paper support by a suitable software and extracting the data, and also suitable to control the whole terminal, at least a printer, memorisation means, wherein, following to the acquisition of the images by the scanner, and the extraction of the information by said processing means, printer obliterates said paper support and sad memorisation means store said information fro each paper support.

Still according to the invention, each one of said plurality of terminals can comprise network connection means, preferably a modem.

Furthermore, according to the invention, said scanner can acquire images on both sides of the paper support.

Always according to the invention, said scanner can comprise optic sensors and dragging means for the support paper during its scanning.

Still according to the invention, said printer can comprise an optic sensor to verify the obliteration.

Preferably, according to the invention, said data support means can comprise a capsule containing flash memories with anti-effraction systems.

Still according to the invention, each one of said plurality of terminals can comprise interface means such as a monitor and/or a display and/or a keyboard and/or a laying device and/or an optic pen.

Preferably, according to the invention, each one of said plurality of terminals can comprise client and/or pharmacist recognition means, preferably a badge and/or electronic identity cards reader.

Furthermore, according to the invention, said server can be connected to a plurality of sub-servers.

The present invention will be now described, for illustrative but not limitative purposes, according to its preferred embodiments, with particular reference to the figures of the enclosed drawings, wherein:
Figure 1 shows a general scheme of the data collection centralised system; an
Figure 2 shows a block diagram for the operation of the operative terminal of the system of figure 1.

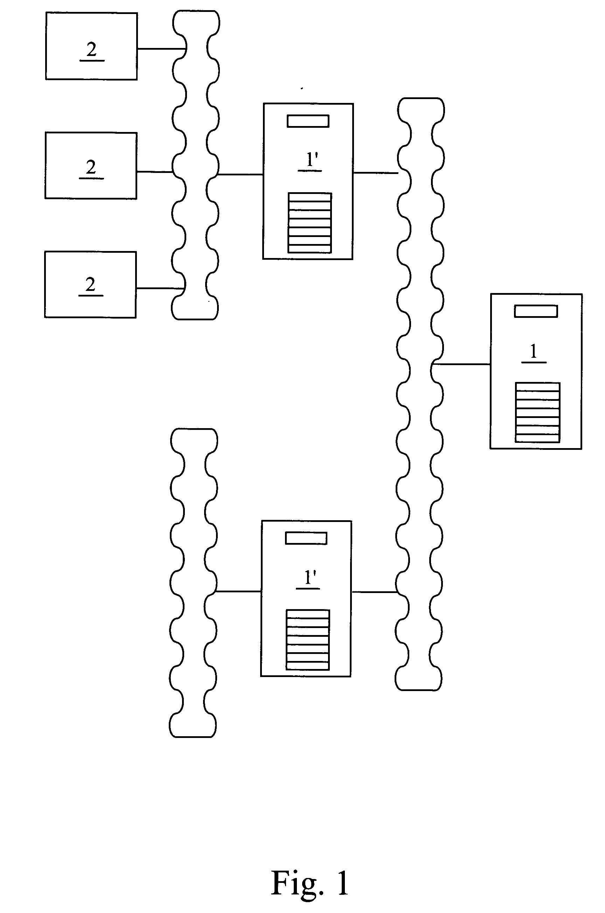

Making reference to figure 1, it is possible to observe the general scheme of the system. Particularly, it is possible to observe a server 1, that can be identified, for example, with the national server, containing the data arriving from all the other regional servers 1' connected with the same. Servers 1' are then network connected with terminals 2, provided with the pharmacies of each region or state.

Network connection is carried out by known systems, such as modem, ISDN, XDSL modem or LAN network. It is further provided that servers can be connected with terminal 2 also when it is switched off. It allows to update control or managing software, downloading inside data of terminal or loading prices and lists of drugs reimbursed by public health in any moment, also when the terminal is switched off.

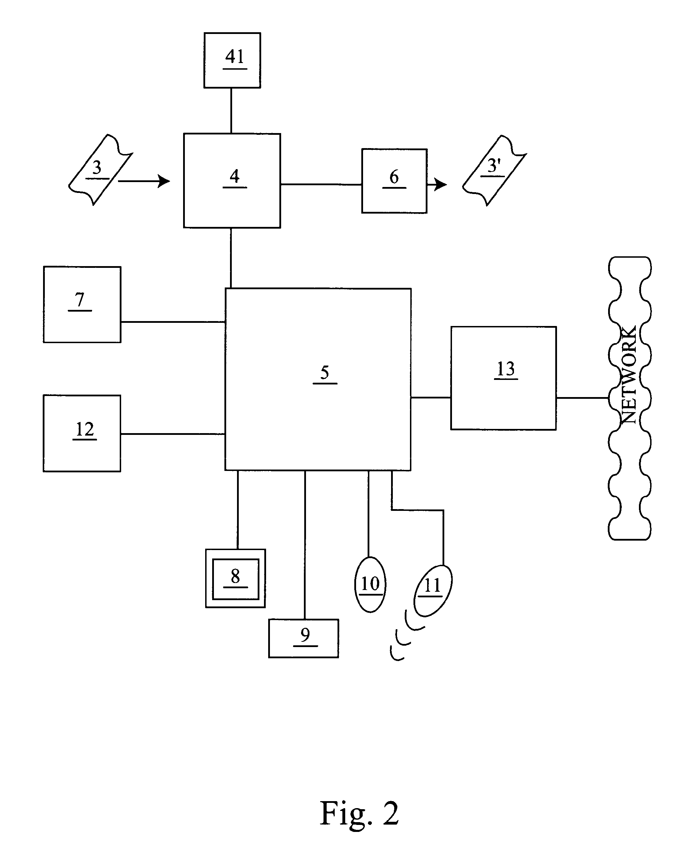

In figure 2 it is shown a block diagram of a terminal 2. in the following, a description of the various parts and of their working will be given. Particularly, it is schematically shown a prescription 3 introduced within the scanner 4. The latter reads both sided of the prescription 3, by optical devices such as CCD (Charged Coupled device) or linear sensors or image contact. More particularly, scanner, during the first dragging, acquires two images, the front part and the rear part, while in the return stroke said optic sensors check the print of the obliteration or the putting of electronic signature, a it will be described in the following. Being the scanner of the dragging type, it is also provided a roll motor 41 for dragging the prescription 3.

The two images (front and rear parts) of the prescription are sent to the processor 5, that can be also a mother board of a personal computer. Processor 5 has, inside, a program allowing to decode the content of a hand written image, converting images into alpha-numeric data. In this way, processor 5 will also read the code number of the Client. Then, in view of the use of electronic identity cards, it is provided a smart-card or badge reader, that can immediately and at the same time provide information about the Client or the pharmacist.

The elaborator 5 carries out a prescription validity control 3, for example checking the serial number of the prescription 3.

After the reading and the validation of the prescription, on the basis of the correspondence of the prescription/client data, prescription is passed to a printer 6. then, optic sensors check the obliteration of the prescription. Obliteration of the prescription 3 by the printer 6 makes useless other attempted improper use of the same prescription 3. Obliterated prescription 3' is ejected and stored by the pharmacist according to the standard way.

A plurality of accessory are connected with the processor, allowing the control and possible manual execution of activities. Among these accessory, they can be mentioned a monitor 8 or display, a keyboard 9, a laying means, such as a mouse 10 and an optic pen 11 for reading, for example, the bar code.

For each processed prescription 3, a new record is created containing the acquired images, decoded data, correction made by the operator, some service fields and control codes; the electronic signature of the operator is applied to this record.

These records are memorised both in the server 1, and within a capsule 12. Capsule 12 is a device containing a Flash memory, presently available with different sizes, up to 512 MB. Capsule 12 is provided with one or more anti-effraction devices, preventing is re-writing. A large amount of records is memorised within said capsule 12, one for each processed "prescription", including the images of the same "prescriptions", as well as some software codes necessary to the working of the apparatus. Capsule will be sized in such a way to contain all the data relevant to the prescription managed during at least one month.

Finally, terminal 2 provides that also a network connection device 13 is connected with the processor 5, such as an internet or LAN network modem.

Periodically, terminal connects with the server 1 under request by the operator or the same server 1, for exchanging data using the cryptography systems necessary to guarantee a high safety and confidential level.

Periodically, or under request of the operator, terminal generates a note summarising the whole activity.

Finally, if all the operation had a positive outcome, a validation and obliteration message appears on the display. In the negative, terminal signals anomalies individuated and the consequent operations to be carried out.

As it can be noted it is possible to extract from server 1 or 1'information relevant to the drug expense, epidemic analysis report, impact analyses report of the measures adopted. From terminals 2 it is possible to extract information such as a list of the regional assisted register and of doctors or of Local Health Organisations, list of free drugs, provisions taken to control the health system.

On the basis of the above specification, it can be noted that the basic feature is that of providing a series of controls allowing to carry out the control of drugs paid by public administration when it occurs the exchange prescription - drug, and not after the selling of the same drug.

Furthermore, terminal can be integrated in a single device, making thus it very versatile.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Centralised data processing and collecting system, on the basis of the reading of the information on a paper support, preferably a medical prescription, comprising
a plurality of terminals suitable to read said paper support, extracting the written information, obliterate said paper support and memorising said information, at least one server for receiving and processing said data stored in said plurality of terminals
data from said plurality of terminals being transmitted to aid server by network connection and/or by physical transfer of data supporting means.

2. System according to claim 1, **characterised in that** each one of said plurality of terminals ca comprise at least a scanner for acquiring images from said paper support, processing means, suitable to elaborate said images of said paper support by a suitable software and extracting the data, and also suitable to control the whole terminal, at least a printer, memorisation means, wherein, following to the acquisition of the images by the scanner, and the extraction of the information by said processing means, printer obliterates said paper support and sad memorisation means store said information fro each paper support.

3. System according to claim 3, **characterised in that** each one of said plurality of terminals comprises network connection means, preferably a modem.

4. System according to claim 2 or 3, **characterised in that** said scanner acquires images on both sides of the paper support.

5. System according to claim 3, **characterised in that** said scanner comprises optic sensors and dragging means for the support paper during its scanning.

6. System according to claim 5, **characterised in that** said optic sensors verify the obliteration.

7. System according to one of the preceding claims 2 - 6, **characterised in that** said data support means comprises a capsule containing flash memories with anti-effraction systems.

8. System according to one of the preceding claims 2 - 7, **characterised in that** each one of said plurality of terminals comprises interface means such as a monitor and/or a display and/or a keyboard and/or a laying device and/or an optic pen.

9. System according to one of the preceding claims 2 - 8, **characterised in that** each one of said plurality of terminals can comprise client and/or pharmacist recognition means, preferably a badge and/or electronic identity cards reader.

10. System according to one of the preceding claims 2 - 7, **characterised in that** said server can be connected to a plurality of sub-servers.

11. System according to each one of the preceding claims, substantially as illustrated and described.
